# EUROPEAN PATENT SPECIFICATION

(11) **EP 2 334 809 B1**
(45) Date of publication and mention of the grant of the patent: **05.11.2014**
(21) Application number: 09736167.9
(22) Date of filing: 29.09.2009
(51) Int. Cl.: C12Q 1/68

(54) **MOLECULAR MARKERS IN PROSTATE CANCER**
MOLEKULARE MARKER BEI PROSTATAKREBS
MARQUEURS MOLÉCULAIRES POUR LE CANCER DE LA PROSTATE

(30) Priority: 01.10.2008 WO PCT/EP2008/008474
(43) Date of publication of application: 22.06.2011
(73) Proprietor: Noviogendix Research B.V., 6525 GA Nijmegen (NL)
(72) Inventor: SMIT, Franciscus, Petrus, NL-6546 RN Nijmegen (NL); SCHALKEN, Jack, A., NL-6524 LH Nijmegen (NL); HESSELS, Daphne, NL-6581 HJ Malden (NL); JANNINK, Sander, Adriaan, NL-6541 NP Nijmegen (NL)
(74) Representative: van Kooij, Adriaan
(86) International application number: PCT/EP2009/062601
(87) International publication number: WO 2010/037735

(56) References cited:
- EP-A- 1 897 940
- WO-A-2006/056766
- WO-A-2006/091776
- WO-A-2007/056049
- US-A1- 2004 009 481
- KOLESAR JILL ET AL: "Evaluation of mRNA by Q-RTPCR and protein expression by AQUA of the M2 subunit of ribonucleotide reductase (RRM2) in human tumors" CANCER CHEMOTHERAPY AND PHARMACOLOGY, vol. 64, no. 1, June 2009 (2009-06), pages 79-86, XP002556111 ISSN: 0344-5704
- FAN HUIZHOU ET AL: "The mammalian ribonucleotide reductase R2 component cooperates with a variety of oncogenes in mechanisms of cellular transformation" CANCER RESEARCH, vol. 58, no. 8, 15 April 1998 (1998-04-15), pages 1650-1653, XP002556112 ISSN: 0008-5472
- FAN HUIZHOU ET AL: "Ribonucleotide reductase R2 component is a novel malignancy determinant that cooperates with activated oncogenes to determine transformation and malignant potential" PROCEEDINGS OF THE NATIONAL ACADEMY OF SCIENCES OF THE UNITED STATES OF AMERICA, vol. 93, no. 24, 1996, pages 14036-14040, XP002556113 ISSN: 0027-8424

## Description

The present invention relates to methods for diagnosing prostate cancer and especially diagnosing low grade (LG) prostate cancer, i.e., individuals with good prognosis; high grade (HG) prostate cancer, i.e., individuals with poor prognosis of primary tumour; PrCa Met, i.e., individuals with poor prognosis and metastasis; and castration resistant prostate cancer (CRPC), i.e., individuals with poor prognosis that are progressive under endocrine therapy and are suffering from aggressive localized disease.

In the Western male population, prostate cancer has become a major public health problem. In many developed countries, it is not only the most commonly diagnosed malignancy, but prostate cancer is also the second leading cause of cancer related deaths in males as well. Because the incidence of prostate cancer increases with age, the number of newly diagnosed cases continues to rise as the life expectancy of the general population increases. In the United States, approximately 193,000 men, and in the European Union, approximately 183,000 men, are newly diagnosed with prostate cancer every year.

Epidemiological studies show that prostate cancer is an indolent disease and more men die with prostate cancer than from it. However, a significant fraction of the tumours behave aggressively and, as a result, approximately 35,800 American men and approximately 80,000 European men die from this disease on an annual basis.

The high mortality rate is a consequence of the fact that there are no effective curative therapeutic options for metastatic prostate cancer. Androgen ablation is generally the treatment of choice in men with metastatic disease. Initially, 70 to 80% of the patients with advanced disease show a response to therapy, but with time the majority of the tumours are observed to become androgen independent, also designated as the castration resistant stage(formarly designated as hormone-refractory stage). As a result, most patients will develop progressive disease.

Currently, there is no effective treatment for the castration resistant stage of prostate cancer. More than 70% of the castration resistant patients suffer from painful bone metastases, which is the major cause of morbidity.

Radical prostatectomy and radiotherapy are curative therapeutic options for prostate cancer, but their curative potential is limited to anatomically localized disease. Early detection of prostate cancer, when the disease is confined to the prostate, is therefore pivotal. Since its discovery more than 20 years ago, prostate specific antigen (PSA) has been the most valuable tool in the detection, staging and monitoring of prostate cancer.

Although widely accepted as a prostate tumour marker, prostate specific antigen (PSA) is known to be prostate tissue- but not prostate cancer-specific. PSA levels have been reported to be increased in men with benign prostatic hyperplasia (BPH) and prostatitis. This substantial overlap in serum PSA values between men with non-malignant prostatic diseases and prostate cancer is the major factor contributing to the limitative use of PSA as a prostate tumour marker.

Moreover, a single reading of PSA cannot be used to differentiate the aggressive tumours from the indolent tumours. Upon detection of serum PSA values of more than 3 ng/ml, the conventional diagnostic approach is the traditional sextant TRUS guided prostate biopsies. However, the low specificity of serum PSA results in a negative biopsy rate of 70 to 80%. In some cases, biopsy specimens may not be representative, also attributing to the failure to detect some cancers, or, in other words, false negative diagnosis.

Currently, most academic centres recommend extension of the diagnostic set to 10 biopsies thereby accepting the risk of diagnosing more indolent cancers. In case of persistent rising serum PSA levels, repeated biopsies are proposed which have at least 10% probability of demonstrating cancer. Moreover, if the combined use of serum PSA, DRE and TRUS biopsy indicates clinically confined cancer, 40% of these men are found to have already extra-capsular disease upon radical prostatectomy.

Therefore, non-invasive molecular tests, capable of identifying those men having an early stage, clinically localized prostate cancer are urgently needed thereby providing through early radical intervention a prolonged survival and quality of life.

Molecular markers identified in tissues can serve as target for new body fluid based molecular tests for prostate cancer. Recent developments in the field of molecular biology have provided tools that have led to the discovery of many new promising biomarkers for prostate cancer. These biomarkers may be instrumental in the development of new tests that have a high specificity in the diagnosis and/or prognosis of prostate cancer.

A suitable biomarker preferably fulfils the following two criteria: 1) it must be reproducible (intra-and inter-institutional) and 2) it must have an impact on clinical management.

Further, for diagnostic purposes, it is important that the biomarkers are tested in terms of tissue-specificity and discrimination potential between prostate cancer, normal prostate and BPH. Furthermore, it can be expected that (multiple) biomarker-based assays enhance the specificity for cancer detection.

Considering the above, there is an urgent need in the art for molecular prognostic biomarkers indicative of the biological behaviour of cancer and clinical outcome.

For the identification of new candidate markers for prostate cancer, it is a perquisite to study expression patterns in malignant as well as non-malignant prostate tissues, preferably in relation to other medical data.

Recent developments in the field of molecular biology have provided tools enabling the assessment of both genomic alterations and proteomic alterations in prostate tumour samples in a comprehensive and rapid manner.

For instance, the identification of different chromosomal abnormalities, like changes in chromosome number, translocations, deletions, rearrangements and duplications in cells, can be studied using fluorescence *in situ* hybridization (FISH) analysis. Also comparative genomic hybridization (CGH) is capable of screening the entire genome for large changes in DNA sequence copy number or deletions larger than 10 mega-base pairs. Differential display analysis, serial analysis of gene expression (SAGE), oligonucleotide arrays and cDNA arrays characterize gene expression profiles. These techniques are often used combined with tissue microarray (TMA) for the identification of genes that play an important role in specific biological processes.

Considering that genetic alterations often result in mutated or altered proteins, the signalling pathways of a cell may become affected. Eventually, this may lead to a growth advantage, or increased survival, of a cancer cell.

Proteomics studies the identification of altered proteins in terms of structure, quantity, and post-translational modifications. Disease-related proteins can be directly sequenced and identified in intact whole tissue sections using the matrix-assisted laser desorption-ionization time-of-flight mass spectrometer (MALDI-TOF). Additionally, surface-enhanced laser desorption-ionization (SELDI)-TOF mass spectroscopy (MS) can provide a rapid protein expression profile from tissue cells and body fluids like serum or urine.

In the last years, these molecular tools have led to the identification of hundreds of genes that are believed to be relevant in the development of prostate cancer. Not only have these findings led to more insight in the initiation, and progression, of prostate cancer, but they have also shown that prostate cancer is a very heterogeneous disease.

Several prostate tumours may occur in the prostate of a single patient due to the multifocal nature of the disease. Each of these tumours can show remarkable differences in gene expression and behaviour associated with varying prognoses. Therefore, in predicting the outcome of the disease, it is more likely that a set of different markers will become of clinical importance.

Biomarkers can be classified into four different prostate cancer-specific events: genomic alterations, prostate cancer-specific biological processes, epigenetic modifications and genes uniquely expressed in prostate cancer.

One of the strongest epidemiological risk factors for prostate cancer is a positive family history. A study of 44,788 pairs of twins in Denmark, Sweden and Finland has shown that 42% of the prostate cancer cases were attributable to inheritance. Consistently higher risk for the disease has been observed in brothers of affected patients compared to the sons of the same patients. This has led to the hypothesis that there is an X-linked or recessive genetic component involved in the risk for prostate cancer.

Genome-wide scans in affected families implicated at least seven prostate cancer susceptibility loci designated as HPC1 (1q24), CAPB (1p36), PCAP (1q42), ELAC2 (17p11), HPC20 (20q13), 8p22-23 and HPCX (Xq27-28). Three candidate hereditary prostate cancer genes have been mapped to these loci, HPC1/2'-5'-oligoadenylate dependent ribonuclease L (RNASEL) on chromosome 1q24-25, macrophage scavenger 1 gene (MSR1) located on chromosome 8p22-23, and HPC2/ELAC2 on chromosome 17p11.

It has been estimated that prostate cancer susceptibility genes probably account for only 10% of hereditary prostate cancer cases. The other 30% of familial prostate cancers are most likely associated with shared environmental factors or more common genetic variants or polymorphisms. Since such variants may occur at high frequencies in the affected population, their impact on prostate cancer risk can be substantial.

Polymorphisms in the genes coding for the androgen-receptor (AR), 5α-reductase type II (SRD5A2), CYP17, CYP3A, vitamin D receptor, PSA, GST-T1, GST-M1, GST-P1, IGF-I, and IGF binding protein 3 have been studied to evaluate whether they are capable of predicting the presence of prostate cancer in patients indicated for prostate biopsies because of PSA levels of more than 3 ng/ml. No associations were found between the androgen receptor, SRD5A2, CYP17, CYP3A4, vitamin D receptor, GST-M1, GST-P1, and IGF binding protein 3 genotypes and prostate cancer risk. Only GST-T1 and IGF-I polymorphisms were found to be modestly associated with prostate cancer risk.

Unlike the adenomatous polyposis coli (APC) gene in familial colon cancer, none of the above prostate cancer susceptibility genes, and loci, is by itself the major cause of the largest portion of prostate cancers.

Epidemiology studies support the idea that most prostate cancers can be attributed to factors as race, life-style, and diet. The role of gene mutations in known oncogenes and tumour suppressor genes is probably very small in primary prostate cancer. For instance, the frequency of p53 mutations in primary prostate cancer is reported to be low but have been observed in almost 50% of advanced prostate cancers.

Screening men for the presence of cancer-specific gene mutations or polymorphisms is time-consuming and costly. Moreover, it is very ineffective in the detection of primary prostate cancers in the general male population. Therefore, it cannot be applied as a prostate cancer screening test.

Mitochondrial DNA is present in approximately 1,000 to 10,000 copies per cell. Because of these quantities, mitochondrial DNA mutations have been used as target for the analysis of plasma and serum DNA from prostate cancer patients. Mitochondrial DNA mutations were detected in three out of three prostate cancer patients having the same mitochondrial DNA mutations in their primary tumour. Different urological tumour specimens have to be studied and larger patient groups are needed to define the overall diagnostic sensitivity of this method.

Critical alterations in gene expression can lead to the progression of prostate cancer. Microsatellite alterations, which are polymorphic repetitive DNA sequences, often appear as loss of heterozygosity (LOH) or as microsatellite instability. Defined microsatellite alterations are known in prostate cancer. The clinical utility so far is deemed neglible. The prime use of whole genome - and SNP arrays is considered to be as powerful discovery tools.

Alterations in DNA, without changing the order of bases in the sequence, often lead to changes in gene expression. These epigenetic modifications are changes like DNA methylation and histone acetylations or deacetylations. Many gene promoters contain GC-rich regions also known as CpG islands. Abnormal methylation of CpG islands results in decreased transcription of the gene into mRNA.

It has been suggested that the DNA methylation status may be influenced in early life by environmental exposures, like nutritional factors or stress, and that this leads to an increased risk for cancer in adults. Changes in DNA methylation patterns have been observed in many human tumors. For detection of promoter hypermethylation, a technique designated as methylation-specific PCR (MSP) is used. In contrast to microsatellite or LOH analysis, this technique requires a tumour to normal ratio of only 0.1-0.001%. This means that using this technique, hypermethylated alleles from tumour DNA can be detected in the presence of 10⁴ -10⁵ excess amounts of normal alleles.

Accordingly, DNA methylation can serve as a useful marker in cancer detection. Recently, there have been many reports on hypermethylated genes in human prostate cancer. Two of these genes are RASSF1A (ras association domain family protein isoform A) and GSTP1.

Hypermethylation of RASSF1A is a common phenomenon in breast cancer, kidney cancer, liver cancer, lung cancer and prostate cancer. In 60-70% of prostate tumours, RASSF1A hypermethylation has been found, showing a clear association with aggressive prostate tumors. No RASSF1A hypermethylation has been detected in normal prostate tissue. These findings suggest that RASSF1A hypermethylation may distinguish the more aggressive tumours from the indolent ones. Further studies are needed to assess its diagnostic value.

The most frequently described epigenetic alteration in prostate cancer is the hypermethylation of the Glutathione S-transferase P1 (GSTP1) promoter. GSTP1 belongs to the cellular protection system against toxic effects and as such this enzyme is involved in the detoxification of many xenobiotics.

GSTP1 hypermethylation has been reported in approximately 6% of the proliferative inflammatory atrophy (PIA) lesions and in 70% of the PIN lesions. It has been shown that some PIA lesions merge directly with PIN and early carcinoma lesions, although additional studies are necessary to confirm these findings. Hypermethylation of GSTP1 has been detected in more than 90% of prostate tumours, whereas no hypermethylation has been observed in BPH and normal prostate tissues.

In another study, hypermethylation of the GSTP1 gene has been detected in 50% of ejaculates from prostate cancer patients but not in men with BPH. Because of the fact that ejaculates are not always easily obtained from prostate cancer patients, hypermethylation of GSTP1 was determined in urinary sediments obtained from prostate cancer patients after prostate massage. In 77% of these sediments cancer could be detected.

Moreover, hypermethylation of GSTP1 has been found in urinary sediments after prostate massage in 68% of patients with early confined disease, 78% of patients with locally advanced disease, 29% of patients with PIN and 2% of patients with BPH. These findings resulted in a specificity of 98% and a sensitivity of 73%. The negative predictive value of this test was 80%, which shows that this assay bears great potential to reduce the number of unnecessary biopsies.

GSTP1 hypermethylation has been detected in 40 to 50% of urinary sediments that were obtained from patients who just underwent prostate biopsies. GSTP1 hypermethylation was detected in urinary sediments of patients with negative biopsies (33%) and patients with atypia or high-grade PIN (67%). Because hypermethylation of GSTP1 has a high specificity for prostate cancer, it suggests that these patients may have occult prostate cancer. This indicates that the test could also be used as indicator for a second biopsy. Other cancer associated genes are also know to be methylated such as APC and Cox 2.

Micro-array studies have been useful and informative to identify genes that are consistently up-regulated or down-regulated in prostate cancer compared to benign prostate tissue. These genes can provide prostate cancer-specific biomarkers and provide insight into the etiology of the disease.

For molecular diagnosis of prostate cancer, genes that are highly up-regulated in prostate cancer compared to low or normal expression in normal prostate tissue are of special interest. Such genes could enable the detection of one tumour cell in a large background of normal cells, and could therefore be applied as a diagnostic marker in prostate cancer detection.

cDNA micro array analysis in the prostate cancer cell line LNCAP has led to the discovery of serine protease TMPRSS2, which was found to be up-regulated by androgens. *In situ* hybridization studies have shown that TMPRSS2 was highly expressed in the basal cells of normal human prostate tissue and in adenocarcinoma cells. Low expression of TMPRSS2 has been found in colon, lung, kidney, and pancreas.

A 492 amino acid protein has been predicted for TMPRSS2. This predicted protein is a type II integral membrane protein, most similar to the hepsin family. These proteins are important for cell growth and maintenance of cell morphology. It is proposed that TMPRSS2 could be an activator of the precursor forms of PSA and hK2 and that TMPRSS2, like other serine proteases, may play a role in prostate carcinogenesis. Since TMPRSS2 has a low prostate cancer-specificity, it cannot be applied in the detection of prostate cancer cells in urinary sediments.

The gene coding for α-methylacyl-CoA racemase (AMACR) on chromosome 5p13 has been found to be consistently up-regulated in prostate cancer. This enzyme plays a critical role in peroxisomal beta oxidation of branched chain fatty acid molecules obtained from dairy and beef. Interestingly, the consumption of dairy and beef has been associated with an increased risk for prostate cancer.

In clinical prostate cancer tissue, a 9-fold over-expression of AMACR mRNA has been found compared to normal prostate tissue. Immunohistochemical (IHC) studies and Western blot analyses have confirmed the up-regulation of AMACR at the protein level. Furthermore it has been shown that 88% of prostate cancer cases and both untreated metastases and castration resistant prostate cancers were strongly positive for AMACR. AMACR expression has not been detected in atrophic glands, basal cell hyperplasia and urothelial epithelium or metaplasia. IHC studies also showed that AMACR expression in needle biopsies had a 97% sensitivity and a 100% specificity for prostate cancer detection.

Combined with a staining for p63, a basal cell marker that is absent in prostate cancer, AMACR greatly facilitated the identification of malignant prostate cells. Its high expression and cancer-cell specificity implicate that AMACR may also be a candidate for the development of molecular probes which may facilitate the identification of prostate cancer using non-invasive imaging modalities.

Using cDNA micro array analysis, it has been shown that hepsin, a type II transmembrane serine protease, is one of the most-differentially over-expressed genes in prostate cancer compared to normal prostate tissue and BPH tissue. Using a quantitative real-rime PCR analysis it has been shown that hepsin is over-expressed in 90% of prostate cancer tissues. In 59% of the prostate cancers this over-expression was more than 10-fold.

Also, there has been a significant correlation between the up-regulation of hepsin and tumour-grade. Further studies will have to determine the tissue-specificity of hepsin and the diagnostic value of this serine protease as a new serum marker. Since hepsin is up-regulated in advanced and more aggressive tumours, it suggests a role as a prognostic tissue marker to determine the aggressiveness of a tumour.

Telomerase, a ribonucleoprotein, is involved in the synthesis and repair of telomeres that cap and protect the ends of eukaryotic chromosomes. The human telomeres consist of tandem repeats of the TTAGGG sequence as well as several different binding proteins. During cell division telomeres cannot be fully replicated and will become shorter. Telomerase can lengthen the telomeres and thus prevents the shortening of these structures. Cell division in the absence of telomerase activity will lead to shortening of the telomeres. As a result, the lifespan of the cells becomes limited and this will lead to senescence and cell death.

In tumour cells, including prostate cancer cells, telomeres are significantly shorter than in normal cells. In cancer cells with short telomeres, telomerase activity is required to escape senescence and to allow immortal growth. High telomerase activity has been found in 90% of prostate cancers and was shown to be absent in normal prostate tissue.

In a small study on 36 specimens, telomerase activity has been used to detect prostate cancer cells in voided urine or urethral washing after prostate massage. This test had a sensitivity of 58% and a specificity of 100%. The negative predictive value of the test was 55%. Although it has been a small and preliminary study, the low negative predictive value indicates that telomerase activity measured in urine samples is not very promising in reducing the number of unnecessary biopsies.

The quantification of the catalytic subunit of telomerase, hTERT, showed a median over-expression of hTERT mRNA of 6-fold in prostate cancer tissues compared to normal prostate tissues. A significant relationship was found between hTERT expression and tumour stage, but not with Gleason score. The quantification of hTERT using real-time PCR showed that hTERT could well discriminate prostate cancer tissues from non-malignant prostate tissues. However, hTERT mRNA is expressed in leukocytes, which are regularly present in body fluids such as blood and urine. This may cause false positivity. As such, quantitative measurement of hTERT in body fluids is not very promising as a diagnostic tool for prostate cancer.

Prostate-specific membrane antigen (PSMA) is a transmembrane glycoprotein that is expressed on the surface of prostate epithelial cells. The expression of PSMA appears to be restricted to the prostate and it has been shown that PSMA is up-regulated in prostate cancer tissue compared to benign prostate tissues. No overlap in PSMA expression has been found between BPH and prostate cancer indicating that PSMA is a promising diagnostic marker.

It has been shown that high PSMA expression in prostate cancer cases correlated with tumour grade, pathological stage, aneuploidy, and biochemical recurrence. Moreover, increased PSMA mRNA expression in primary prostate cancers and metastasis correlated with PSMA protein over-expression. Its clinical utility as a diagnostic or prognostic marker for prostate cancer has been hindered by the lack of a sensitive immunoassay for this protein.

However, a combination of ProteinChip arrays and SELDI-TOF MS has provided the introduction of a protein biochip immunoassay for the quantification of serum PSMA. It was shown that the average serum PSMA levels for prostate cancer patients were significantly higher compared to those of men with BPH and healthy controls. These findings implicate a role for serum PSMA to distinguish men with BPH from prostate cancer patients, but further studies will have to assess its diagnostic value.

RT-PCR studies have shown that PSMA in combination with its splice variant PSMA' could be used as a prognostic marker for prostate cancer. In the normal prostate PSMA' expression is higher than PSMA expression. In prostate cancer tissues the PSMA expression is more dominant. Therefore, the ratio of PSMA over PSMA' is highly indicative for disease progression. Designing a quantitative PCR analysis which discriminates between the two PSMA forms could yield another application for PSMA in diagnosis and prognosis of prostate cancer.

Delta-catenin (p120/CAS), an adhesive junction-associated protein, has been shown to be highly discriminative between BPH and prostate cancer. *In situ* hybridization studies showed the highest expression of δ-catenin transcripts in adenocarcinoma of the prostate and low to no expression in BPH tissue. The average over-expression of δ-catenin in prostate cancer compared to BPH is 15.7 fold.

Both quantitative PCR and *in situ* hybridization analysis could not demonstrate a correlation between δ-catenin expression and Gleason scores. Further studies are needed to assess the tissue-specificity and diagnostic value of δ-catenin, but it is clear that it has limitations when used as a prognostic marker for prostate cancer.

DD3^{PCA3} has been identified using differential display analysis. DD3^{PCA3} was found to be highly over-expressed in prostate tumours compared to normal prostate tissue of the same patient using Northern blot analysis. Moreover, DD3^{PCA3} was found to be strongly over-expressed in more than 95% of primary prostate cancer specimens and in prostate cancer metastasis. Furthermore, the expression of DD3^{PCA3} is restricted to prostatic tissue, i.e., no expression has been found in other normal human tissues.

The gene encoding for DD3^{PCA3} is located on chromosome 9q21.2. The DD3^{PCA3} mRNA contains a high density of stop-codons. Therefore, it lacks an open reading frame resulting in a non-coding RNA. Recently, a time-resolved quantitative RT-PCR assay (using an internal standard and an external calibration curve) has been developed. The accurate quantification power of this assay showed a median 66-fold up-regulation of DD3^{PCA3} in prostate cancer tissue compared to normal prostate tissue. Moreover, a median-up-regulation of 11-fold was found in prostate tissues containing less than 10% of prostate cancer cells. This indicated that DD3^{PCA3} was capable to detect a small number of tumour cells in a large background of normal cells.

This hypothesis has been tested using the quantitative RT-PCR analysis on voided urine samples. PSA mRNA expression was shown to be relatively constant in normal prostate cells and only a weak down-regulation (~1.5-fold) of PSA expression has been reported in prostate cancer cells. Therefore, PSA mRNA has been used as a 'housekeeping gene' to correct for the number of prostate cells present in urinary sediments. These urine samples were obtained after extensive prostate massage from a group of 108 men who were indicated for prostate biopsies based on a total serum PSA value of more than 3 ng/ml. This test had 67% sensitivity and 83% specificity using prostatic biopsies as a gold-standard for the presence of a tumour. Furthermore, this test had a negative predictive value of 90%, which indicates that the quantitative determination of DD3^{PCA3} transcripts in urinary sediments obtained after extensive prostate massage bears great potential in the reduction of the number of invasive TRUS guided biopsies in this population of men.

The tissue-specificity and the high over-expression in prostate tumours indicate that DD3^{PCA3} is the most prostate cancer-specific gene described so far. Therefore, validated DD3^{PCA3} assays could become valuable in the detection of disseminated prostate cancer cells in serum or plasma. Multicenter studies using the validated DD3^{PCA3} assay can provide the first basis for the molecular diagnostics in clinical urological practice.

Modulated expression of cytoplasmic proteins HSP-27 and members of the PKC isoenzyme family, particularly PKC-β and PKC-ε, have been correlated with prostate cancer progression.

Modulation of expression has clearly identified those cancers that are aggressive - and hence those that may require urgent treatment, irrespective of their morphology. Although not widely employed, antibodies to these proteins are authenticated, are available commercially, and are straightforward in their application and interpretation, particularly in conjunction with other reagents as double-stained preparations.

The significance of this group of markers is that they accurately distinguish prostate cancers of aggressive phenotype. Modulated in their expression by invasive cancers, when compared to non-neoplastic prostatic tissues, those malignancies which express either HSP27 or PKCβ at high level invariably exhibit a poor clinical outcome. The mechanism of this association warrants elucidation and validation.

E2F transcription factors, including E2F3 located on chromosome 6p22, directly modulate expression of EZH2. Overexpression of the EZH2 gene has been important in development of human prostate cancer.

EZH2 was identified as a gene overexpressed in castration resistant and metastatic prostate cancer and showed that patients with clinically localized prostate cancers that express EZH2 have a worse progression than those who do not express the protein.

Using tissue micro arrays, expression of high levels of nuclear E2F3 occurs in a high proportion of human prostate cancers but is a rare event in non-neoplastic prostatic epithelium. These data, together with other published information, suggested that the pRB-E2F3-EZH2 control axis may have a crucial role in modulating aggressiveness of individual human prostate cancers.

Kolesar et al. (2009) discloses base-line RRM2 protein and gene expression in tumors of patients receiving 3-AP. Tumor blocks from patients enrolled in phase I and phase II clinical studies using 3-AP, were evaluated for RRM2 expression by quantitative real time polymerase chain reaction and automated quantitative analysis. The authors conclude that both RRM2 gene and protein expression vary by tumor type in baseline tumor samples and that evaluating baseline RRM2 could predict tumors sensitive to 3-AP.

The prime challenge for molecular diagnostics is the identification of clinically insignificant prostate cancer, i.e., separate the biologically aggressive cancers from the indolent tumours. Furthermore, markers predicting and monitoring the response to treatment are urgently needed.

In current clinical settings of over diagnosis and over treatment become more and more manifest, further underlining the need for biomarkers that are capable of providing an accurate identification of the patients that do not- and do- need treatment.

The use of AMACR immunohistochemistry is widely used in the identification of malignant processes in the prostate thereby contributing to the diagnosis of prostate cancer. Unfortunately, the introduction of molecular markers on tissue as prognostic tool has not been validated for any of the markers discussed.

Experiences over the last two decades have revealed the practical and logistic complexity in translating molecular markers into clinical use. Several prospective efforts, taking into account these issues, are currently ongoing to establish clinical utility of a number of markers. Clearly, tissue biorepositories of well documented specimens, including clinical follow up data, play a pivotal role in the validation process.

Novel body fluid tests based on GSTP1 hypermethylation and the gene DD3^{PCA3}, which is highly over-expressed in prostate cancer, enabled the detection of prostate cancer in non-invasively obtained body fluids such as urine or ejaculates.

The application of new technologies has shown that a large number of genes are up-regulated in prostate cancer. For non-invasive screening tests only those genes will be important that are over-expressed in more than 95% of prostate cancer tissues compared to normal prostate or BPH.

Moreover, the up-regulation of these genes in cancer should be more than 10% in prostate cancer compared to normal prostate to enable the detection of a single prostate cancer cell in a large background of normal cells in body fluids such as urine or ejaculates.

Although the markers outlined above, at least partially, address the need in the art for tumour markers, and especially prostate tumour markers, there is a continuing need for reliable (prostate) tumour markers and especially markers indicative of the clinical course and outcome of the disease.

It is an object of the present invention, amongst other objects, to meet at least partially, if not completely, the above need in the art, i.e., the provision of tumour markers providing a reliable identification of prostate cancer in a tissue specimen, and especially a reliable predictive value of the clinical course and outcome of the disease. Such tumour markers will provide a tool aiding a trained physician to decide on a suitable treatment protocol of individuals diagnosed either using tumour markers, or any other indication, with prostate cancer.

According to the present invention, the above object, amongst other objects, is met by the provision of a novel tumour marker in methods as outlined in the appended claims.

Specifically, the above object, amongst other objects, is met by a method for establishing the presence, or absence, of prostate cancer in a human individual comprising:
a) determining the mRNA expression of one or more genes chosen from the group consisting of RRM2 in a sample originating from said human individual;
b) establishing up regulation of expression of said gene as compared to expression of said gene in a sample originating from said human individual not comprising prostate tumour cells or prostate tumour tissue, or from an individual not suffering from prostate cancer; and
c) establishing the presence, or absence, of prostate cancer based on the established up-regulation of said gene.

According to the present invention establishing the presence, or absence, of prostate cancer preferably comprises diagnosis, prognosis and/or prediction of disease survival.

According to the present invention, expression analysis comprises establishing an increased expression of a gene as compared to expression of said gene in a sample originating from said human individual not comprising prostate tumour cells or prostate tumour tissue, or from an individual not suffering from prostate cancer. In other words, an increased expression of a gene according to the present invention is a measure of gene expression relative to a non-disease standard. Establishing an increased expression of RRM2 , as compared to expression of the gene under non-prostate cancer conditions, allows establishing the presence, or absence, of prostate cancer, preferably diagnosis, prognosis and/or prediction of disease survival, according to the present invention.

RRM2: Ribonucleotide reductase (RNR) plays an essential role in ribonucleotide reduction that is required for DNA synthesis and repair. RNR consists of two subunits: RRM1 and RRM2. The activity of RNR, and therefore DNA synthesis and cell proliferation, is controlled during the cell cycle by the synthesis and degradation of RRM2 subunit.

According to the present method, determining the expression comprises determining mRNA expression of said one or more genes.

Expression analysis based on mRNA is generally known in the art and routinely practiced in diagnostic labs world-wide. For example, suitable techniques for mRNA analysis are Northern blot hybridisation and amplification based techniques such as PCR, and especially real time PCR, and NASBA.

According to a particularly preferred embodiment, expression analysis comprises high-throughput DNA array chip analysis not only allowing the simultaneous analysis of multiple samples but also automatic analysis processing.

Disclosed is determining the expression comprises determining protein levels of the genes. Suitable techniques are, for example, matrix-assisted laser desorption-ionization time-of-flight mass spectrometer (MALDI-TOF) based techniques, ELISA and/or immunohistochemistry.

According to the present invention, the present method is preferably further carried out using expression analysis of genes chosen from the group consisting of HOXC6, sFRP2, HOXD10, RORB, TGM4, and SNAI2.

According to a particularly preferred embodiment, the present method is carried out by further expression analysis of HOXC6, sFRP2, HOXD10, RORB, TGM4, and SINAI2.

Preferably, the present presence, or absence, of prostate cancer in a human individual further comprises identification, establishing and/or diagnosing low grade PrCa (LG), high grade PrCa (HG), PrCa Met and/or CRPC.

LG indicates low grade PrCa (Gleason Score equal or less than 6) and represent patients with good prognosis. HG indicates high grade PrCa (Gleason Score of 7 or more) and represents patients with poor prognosis. PrCa Met represents patients with poor prognosis. Finally, CRPC indicates castration resistant prostate cancer and represents patients with aggressive localized disease.

According to a particularly preferred embodiment of the present method, the present invention provides identification, establishing and/or diagnosing CRPC.

Considering the diagnostic value of the present genes as bio- or molecular markers for prostate cancer, disclosed is the use of expression analysis of one or more genes selected from the group consisting HOXC6, sFRP2, HOXD10, RORB, RRM2, TGM4, and SNAI2 for establishing the presence, or absence, of prostate cancer in a human individual.

Also considering the diagnostic value of the present genes as bio- or molecular markers for prostate cancer, disclosed is a kit of parts for establishing the presence, or absence, of prostate cancer in a human individual comprising:
- expression analysis means for determining the expression of one or more genes chosen from the group consisting of HOXC6, sFRP2, HOXD10, RORB, RRM2, TGM4, and SNAI2;
- instructions for use.

The kit of parts can comprise mRNA expression analysis means, preferably suitable for expression analysis by, for example, PCR, rtPCR and/or NASBA.

The kit of parts can comprise means for expression analysis of two or more, three or more, four or more, five or more, six ore more, or seven of the present genes.

In the present description, reference is made to genes suitable as bio- or molecular markers for prostate cancer by referring to their arbitrarily assigned names. Although the skilled person is readily capable of identifying and using the present genes based on the indicated names, the appended figures provide the cDNA sequence of these genes as their accession number, thereby allowing the skilled person to develop expression analysis assays based on analysis techniques commonly known in the art. Such analysis techniques can, for example, be based on the genomic sequence of the gene, the provided cDNA or amino acid sequences. This sequence information can either be derived from the provided sequences, or can be readily obtained from the public databases, for example by using the provided accession numbers.

The present invention will be further elucidated in the following Examples.

In the Examples, reference is made to figures, wherein:

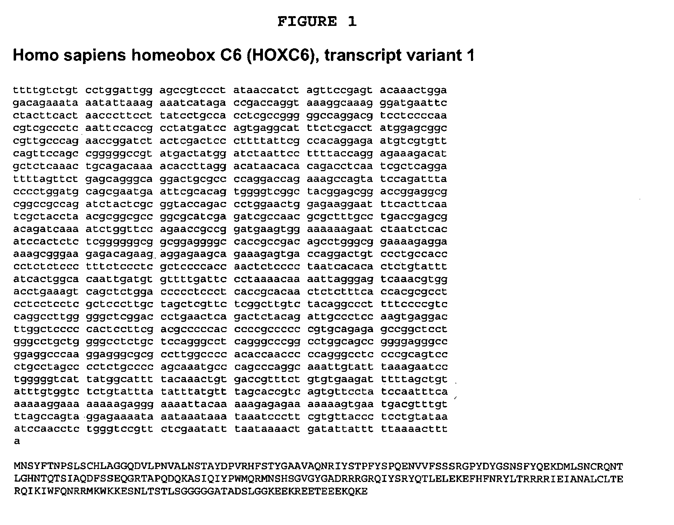

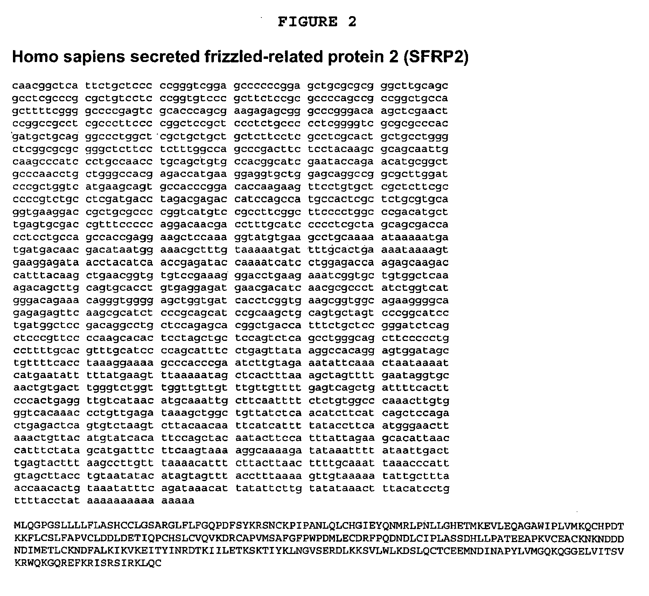

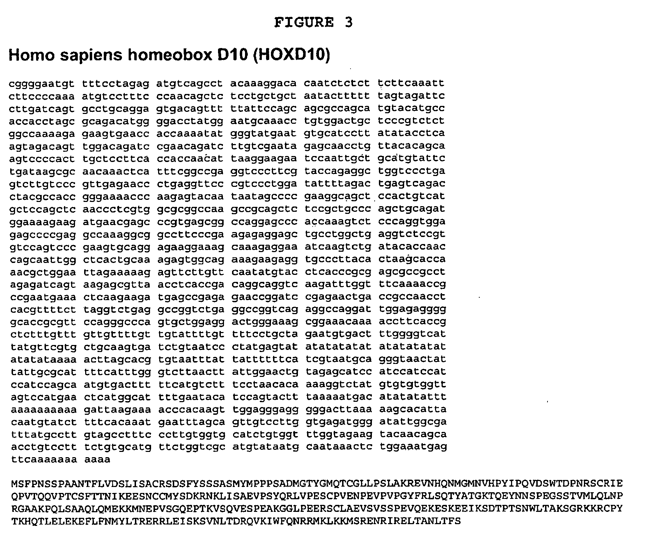

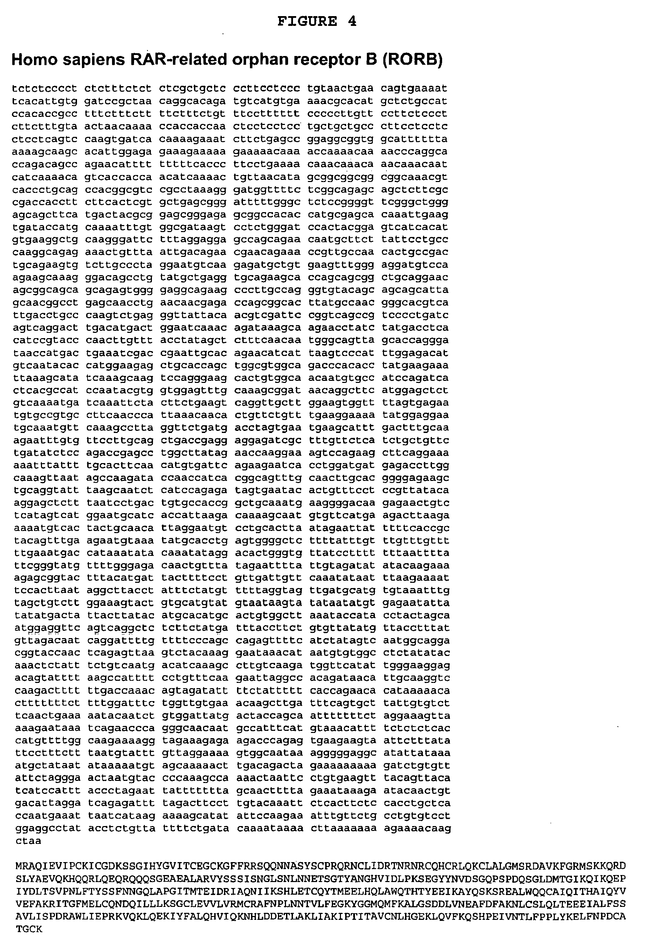

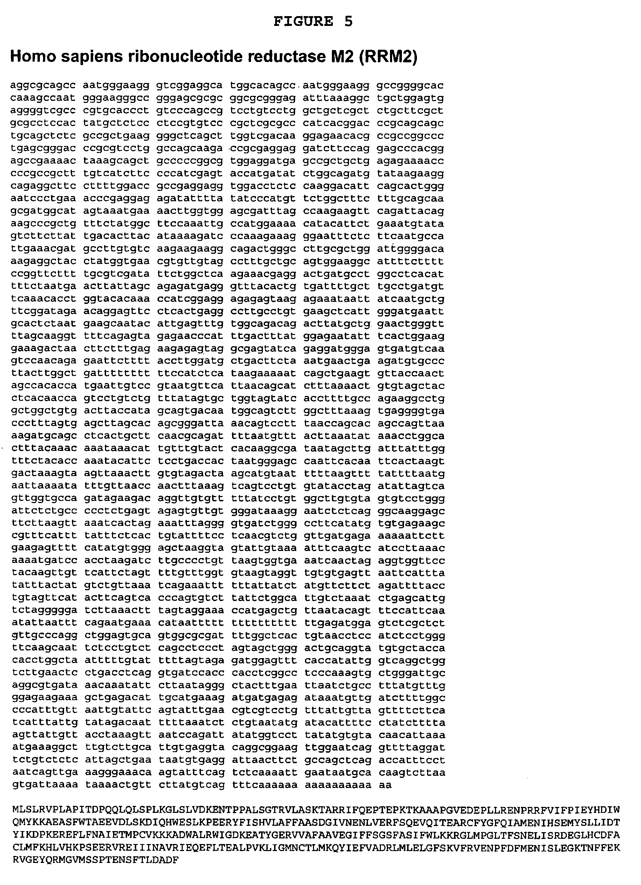

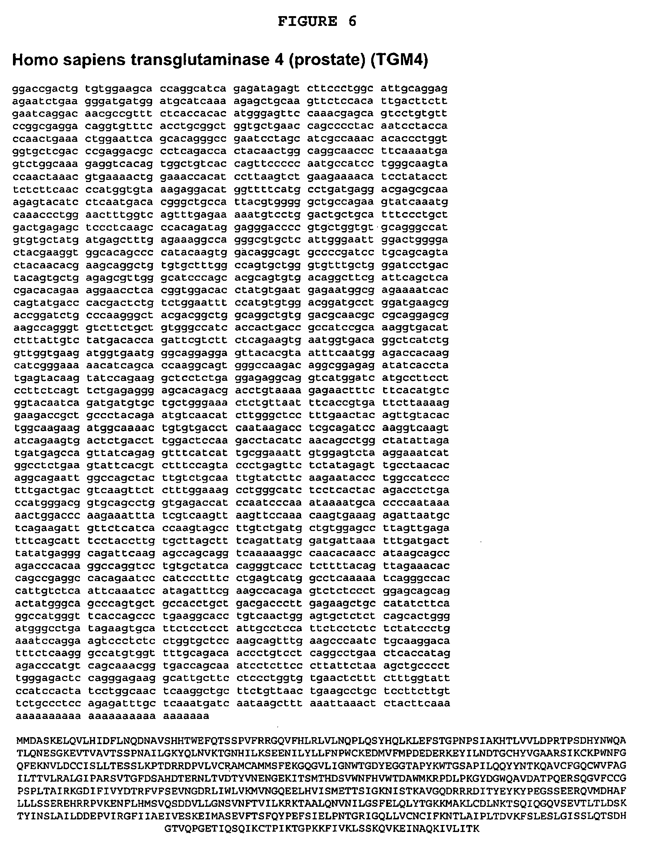

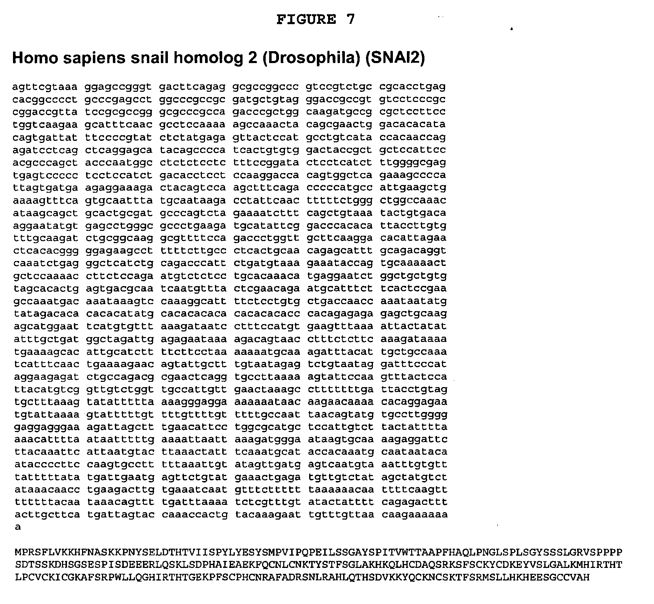

- **Figures 1-7:b**: show the cDNA and amino acid sequences of the HOXC6 gene (NM_004503.3, NP_004494.1); the SFRP2 gene (NM_003013.2, NP_003004.1); the HOXD10 gene (NM_002148.3, NP_002139.2); the RORB gene (NM_006914.3, NP_008845.2); the RRM2 gene (NM_001034.2, NP_001025.1); the TGM4 gene (NM_003241.3, NP_003232.2); and the SNAI2 gene (NM_003068.3, NP_003059.1, respectively;

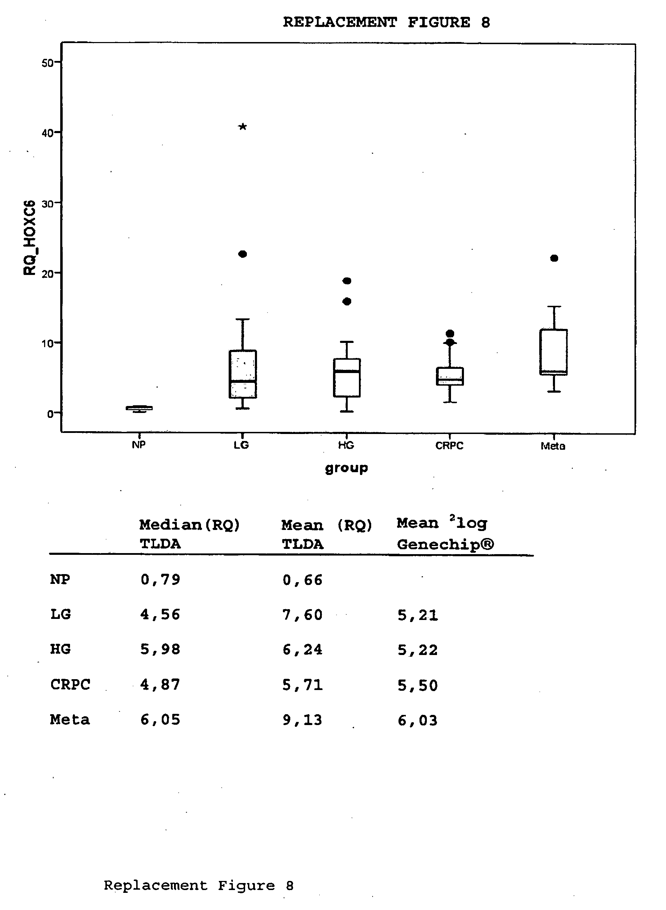

- **Figures 8-14:**: show boxplot TLDA data based on group LG (low grade), HG (high grade), CRPC (castration resistant) and PrCa Met (prostate cancer metastasis) expression analysis of HOXC6 gene (NM_004503.3); the SFRP2 gene (NM_003013.2); the HOXD10 gene (NM_002148.3); the RORB gene (NM_006914.3,); the RRM2 gene (NM_001034.2); the TGM4 gene (NM_003241.3); and the SNAI2 gene (NM_003068.3), respectively. NP indicates no prostate cancer, i.e., normal or standard expression levels.

### EXAMPLES

### Example 1

To identify markers for aggressive prostate cancer, the gene expression profile (GeneChip® Human Exon 1.0 ST Array, Affymetrix )of samples from patients with prostate cancer in the following categories were used:
- LG: low grade PrCa (Gleason Score equal or less than 6). This group represents patients with good prognosis;
- HG: high grade PrCa (Gleason Score of 7 or more). This group represents patients with poor prognosis; sample type, mRNA from primary tumor;
- PrCa Met. This group represents patients with poor prognosis; sample type; mRNA from PrCa metastasis;
- CRPC: castration resistant prostate cancer; mRNA from primary tumor material from patients that are progressive under endocrine therapy. This group represents patients with aggressive localized disease.

The expression analysis is performed according to standard protocols. Briefly, from patients with prostate cancer (belonging to one of the four previously mentioned categories) tissue was obtained after radical prostatectomy or TURP. The tissues were snap frozen and cryostat sections were H.E. stained for classification by a pathologist.

Tumor areas were dissected and total RNA was extracted with TRIzol (Invitrogen, Carlsbad, CA, USA) following manufacturer's instructions. The total RNA was purified with the Qiagen RNeasy mini kit (Qiagen, Valencia, CA, USA). Integrity of the RNA was checked by electrophoresis using the Agilent 2100 Bioanalyzer.

From the purified total RNA, 1 µg was used for the GeneChip Whole Transcript (WT) Sense Target Labeling Assay (Affymetrix, Santa Clara, CA, USA). According to the protocol of this assay, the majority of ribosomal RNA was removed using a RiboMinus Human/Mouse Transcriptome Isolation Kit (Invitrogen, Carlsbad, CA, USA). Using a random hexamer incorporating a T7 promoter, double-stranded cDNA was synthesized. Then cRNA, was generated from the double-stranded cDNA template through an in-vitro transcription reaction and purified using the Affymetrix sample clean-up module. Single-stranded cDNA was regenerated through a random-primed reverse transcription using a dNTP mix containing dUTP. The RNA was hydrolyzed with RNase H and the cDNA was purified. The cDNA was then fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide.

5.5 µg of the fragmented, biotinylated cDNA was added to a hybridization mixture, loaded on a Human Exon 1.0 ST GeneChip and hybridized for 16 hours at 45 °C and 60 rpm.

Using the GeneChip® Human Exon 1.0 ST Array (Affymetrix), genes are indirectly measured by exons analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using an Affymetrix GeneChip Scanner 3000, generating CEL files for each array.

Exon-level expression values were derived from the CEL file probe-level hybridization intensities using the model-based RMA algorithm as implemented in the Affymetrix Expression ConsoleTM software. RMA (Robust Multiarray Average) performs normalization, background correction and data summarization. Differentially expressed genes between conditions are calculated using Anova (ANalysis Of Variance), a T-test for more than two groups.

The target identification is biased since clinically well defined risk groups were analyzed. The markers are categorized based on their role in cancer biology. For the identification of markers the PrCa Met group is compared with 'HG' and 'LG'.

Based on the expression analysis obtained, biomarkers were identified based on 30 tumors; the expression profiles of the biomarkers are provided in **Table 1.**

**Table 1: Expression characteristics of 7 targets characterizing the aggressive metastatic phenotype of prostate cancer based on the analysis of 30 well annotated specimens**

| **Gene name** | **Gene assignment** | **Expression in PrCa Met** | **Met-LG** | **Rank** | **Met-HG** | **Rank** | **Met-CRPC** |
|---|---|---|---|---|---|---|---|
| PTPR | NM_003625 | Up | 15.89 | 4 | 8.28 | 4 | 11.63 |
| EPHA6 | NM_001080448 | Up | 15.35 | 5 | 9.25 | 2 | 8.00 |
| Plakophilin 1 | NM_000299 | Up | 5.28 | 28 | 4.92 | 8 | 5.46 |
| HOXC6 | NM_004503 | Up | 5.35 | 27 | 3.34 | 43 | 3.51 |
| HOXD3 | NM_006898 | Up | 1.97 | 620 | 2.16 | 238 | 1.40 |
| sFRP2 | NM_003013 | Down | -6.06 | 102 | -13.93 | 15 | -3.53 |
| HOXD10 | NM_002148 | Down | -3.71 | 276 | -3.89 | 238 | -5.28 |

### Example 2

The protocol of example 1 was repeated on a group of 70 specimens. The results obtained are presented in **Table 2.**

**Table 2: Expression characteristics of 7 targets validated in the panel of 70 tumors**

| **Gene name** | **Gene assignment** | **Expression in PrCa met** | **Met-LG** | **Rank** | **Met-HG** | **Rank** | **Met-CRPC** | **Rank** |
|---|---|---|---|---|---|---|---|---|
| PTPR | NM_003625 | Up | 6,92 | 1 | 2,97 | 11 | 3,66 | 2 |
| EPHA6 | NM_001080448 | Up | 4,35 | 4 | 3,97 | 3 | 3,18 | 3 |
| Plakophilin 1 | NM_000299 | Up | 3,18 | 12 | 4,00 | 2 | 4,11 | 5 |
| HOXC6 | NM_004503 | Up | 1,77 | 271 | 1,75 | 208 | 1,44 | 6 |
| HOXD3 | NM_006898 | Up | 1,62 | 502 | 1,66 | 292 | 1,24 | 7 |
| sFRP2 | NM_003013 | Down | -6,28 | 46 | -10,20 | 10 | -5,86 | 1 |
| HOXD10 | NM_002148 | Down | -2,48 | 364 | -2,55 | 327 | -2,46 | 4 |

As can be clearly seen in **Tables 1** and **2,** an up regulation of expression of PTPR, EPHA6, Plakophilin 1, HOXC6 **(****Figure 1****)** and HOXD3 was associated with prostate cancer. Further, as can be clearly seen in **Tables 1** and **2,** a down-regulation of expression of sFRP2 **(****Figure 2****)** and HOXD10 **(****Figure 3****)** was associated with prostate cancer.

Considering the above results obtained in 70 tumour samples, the expression data clearly demonstrates the suitability of these genes as bio- or molecular marker for the diagnosis of prostate cancer.

### Example 3

Using the gene expression profile (GeneChip® Human Exon 1.0 ST Array, Affymetrix) on 70 prostate cancers several genes were found to be differentially expressed in low grade and high grade prostate cancer compared with prostate cancer metastasis and castration resistant prostate cancer (CRPC). Together with several other in the GeneChip® Human Exon 1.0 ST Array differentially expressed genes, the expression levels of these genes were validated using the TaqMan® Low Density arrays (TLDA, Applied Biosystems). In **Table 3** an overview of the validated genes is shown.

**Table 3: Gene expression assays used for TLDA analysis**

| **Symbol** | **Gene description** | **Accession number** | **Amplicon size** |
|---|---|---|---|
| AMACR | alpha-methylacyl-CoA racemase | NM_014324 | 97-141 |
| B2M | Beta-2-microglobulin | NM_004048 | 64-81 |
| CYP4F8 | cytochrome P450, family 4, subfamily F | NM_007253 | 107 |
| CDH1 | E-Cadherin | NM_004360 | 61-80 |
| EPHA6 | ephrin receptor A6 | NM_001080448 | 95 |
| ERG | v-ets erythroblastosis virus E26 oncogene homolog | NM_004449 | 60-63 |
| ETV1 | ets variant 1 | NM_004956 | 74-75 |
| ETV4 | ets variant 4 | NM_001986 | 95 |
| ETV5 | ets variant 5 | NM_004454 | 70 |
| FASN | fatty acid synthase | NM_004104 | 144 |
| FOXD1 | forkhead box D1 | NM_004472 | 59 |
| HOXC6 | homeobox C6 | NM_004503 | 87 |
| HOXD3 | homeobox D3 | NM_006898 | 70 |
| HOXD10 | homeobox D10 | NM_002148 | 61 |
| HPRT | hypoxanthine phosphoribosyltransferase 1 | NM_000194 | 72-100 |
| HSD17B6 | hydroxysteroid (17-beta) dehydrogenase 6 homolog | NM_003725 | 84 |
| CDH2 | N-cadherin (neuronal) | NM_001792 | 78-96 |
| CDH11 | OB-cadherin (osteoblast) | NM_001797 | 63-96 |
| PCA3 | prostate cancer gene 3 | AF103907 | 80-103 |
| PKP1 | Plakophilin 1 | NM_000299 | 71-86 |
| KLK3 | prostate specific antigen | NM_001030047 | 64-83 |
| PTPR | protein tyrosine phosphatase, receptor type, f polypeptide | NM_003625 | 66 |
| RET | ret proto-oncogene | NM_020975 | 90-97 |
| RORB | RAR-related orphan receptor B | NM_006914 | 66 |
| RRM2 | ribonucleotide reductase M2 | NM_001034 | 79 |
| SFRP2 | secreted frizzled-related protein 2 | NM_003013 | 129 |
| SGP28 | specific granule protein (28 kDa)/ cysteine-rich secretory protein 3 CRISP3 | NM_006061 | 111 |
| SNAI2 | snail homolog 2 SNAI2 | NM_003068 | 79-86 |
| SNAI1 | snail homolog 1 Snai 1 | NM_005985 | 66 |
| SINK1 | serine peptidase inhibitor, Kazal type 1 | NM_003122 | 85 |
| TGM4 | transglutaminase 4 (prostate) | NM_003241 | 87-97 |
| TMPRSS2 | transmembrane protease, serine 2 | NM_005656 | 112 |
| TWIST | twist homolog 1 | NM_000474 | 115 |

Prostate cancer specimens in the following categories were used (see also **Table 4**):
- Low grade prostate cancer (LG): tissue specimens from primary tumors with a Gleason Score ≤6 obtained after radical prostatectomy. This group represents patients with a good prognosis.
- High grade prostate cancer (HG): tissue specimens from primary tumors with a Gleason Score ≥7 obtained after radical prostatectomy. This group represents patients with poor prognosis.
- Prostate cancer metastases: tissue specimens are obtained from positive lymfnodes after LND or after autopsy. This group represents patients with poor prognosis
- Castration resistant prostate cancer (CRPC): tissue specimens are obtained from patients that are progressive under endocrine therapy and who underwent a transurethral resection of the prostate (TURP).
All tissue samples were snap frozen and cryostat sections were stained with hematoxylin and eosin (H.E.). These H.E.-stained sections were classified by a pathologist.

Tumor areas were dissected. RNA was extracted from 10 µm thick serial sections that were collected from each tissue specimen at several levels. Tissue was evaluated by HE-staining of sections at each level and verified microscopically. Total RNA was extracted with TRIzol® (Invitrogen, Carlsbad, CA, USA) according to the manufacturer's instructions. Total RNA was purified using the RNeasy mini kit (Qiagen, Valencia, CA, USA). RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg DNase-treated total RNA was reverse transcribed using SuperScript™ II Reverse Transcriptase (Invitrogen) in a 37.5 µl reaction according to the manufacturer's protocol. Reactions were incubated for 10 minutes at 25°C, 60 minutes at 42°C and 15 minutes at 70°C. To the cDNA, 62.5 µl milliQ was added.

Gene expression levels were measured using the TaqMan® Low Density Arrays (TLDA; Applied Biosystems). A list of assays used in this study is given in Table 3. Of the individual cDNAs, 3 µl is added to 50 µl Taqman® Universal Probe Master Mix (Applied Biosystems)and 47 µl milliQ. One hundred µl of each sample was loaded into 1 sample reservoir of a TaqMan® Array (384-Well Micro Fluidic Card) (Applied Biosystems). The TaqMan® Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of hypoxanthine phosphoribosyltransferase 1 (HPRT) (endogenous control gene). Furthermore, gene expression values were calculated based on the comparative threshold cycle (Ct) method, in which a normal prostate RNA sample was designated as a calibrator to which the other samples were compared.

For the validation of the differentially expressed genes found by the GeneChip® Human Exon 1.0 ST Array, 70 prostate cancer specimen were used in TaqMan® Low Density arrays (TLDAs). In these TLDAs, expression levels were determined for the 33 genes of interest. The prostate cancer specimens were put in order from low Gleason scores, high Gleason scores, CRPC and finally prostate cancer metastasis. Both GeneChip® Human Exon 1.0 ST Array and TLDA data were analyzed using scatter- and box plots.

In the first approach, scatterplots were made in which the specimens were put in order from low Gleason scores, high Gleason scores, CRPC and finally prostate cancer metastasis. In the second approach, clinical follow-up data were included. The specimens were categorized into six groups: prostate cancer patients with curative treatment, patients with slow biochemical recurrence (after 5 years or more), patients with fast biochemical recurrence (within 3 years), patients that became progressive, patients with CRPC and finally patients with prostate cancer metastasis. After analysis of the box- and scatterplots using both approaches, a list of suitable genes indicative for prostate cancer and the prognosis thereof was obtained **(Table 4,** **Figures 8-14****).**

**Table 4: List of genes identified**

| **Symbol** | **Gene description** | **Accession number** | **Amplicon size** |
|---|---|---|---|
| HOXC6 | homeobox C6 | NM_004503 | 87 |
| SFRP2 | secreted frizzled-related protein 2 | NM_003013 | 129 |
| HOXD10 | homeobox D10 | NM_002148 | 61 |
| RORB | RAR-related orphan receptor B | NM_006914 | 66 |
| RRM2 | ribonucleotide reductase M2 | NM_001034 | 79 |
| TGM4 | transglutaminase 4 (prostate) | NM_003241 | 87-97 |
| SNAI2 | snail homolog 2 SNAI2 | NM_003068 | 79-86 |

HOXC6 (**Figure 8**): The present GeneChip® Human Exon 1.0 ST Array data showed that HOXC6 was upregulated in prostate cancer metastases compared with primary high and low grade prostate cancers. Validation experiments using TaqMan® Low Density arrays confirmed this upregulation. Furthermore, HOXC6 was found to be upregulated in all four groups of prostate cancer compared with normal prostate. Therefore, HOXC6 has diagnostic potential.

Using clinical follow-up data, it was observed that all patients with progressive disease and 50% of patients with biochemical recurrence within 3 years after initial therapy had a higher upregulation of HOXC6 expression compared with patients who had biochemical recurrence after 5 years and patients with curative treatment. The patients with biochemical recurrence within 3 years after initial therapy who had higher HOXC6 expression also had a worse prognosis compared with patients with lower HOXC6 expression. Therefore, HOXC6 expression is correlated with prostate cancer progression.

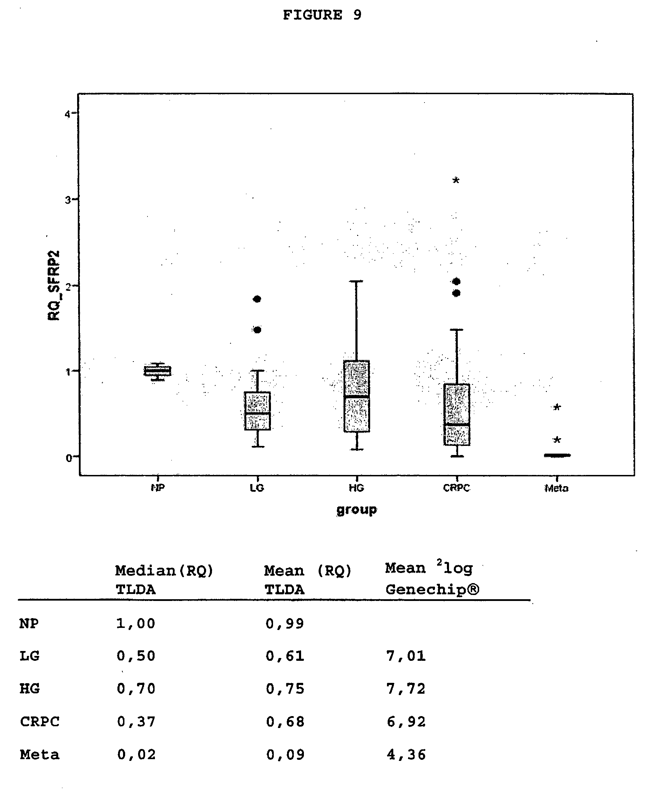

SFRP2 (**Figure 9**): The present GeneChip® Human Exon 1.0 ST Array data showed that SFPR2 was downregulated in prostate cancer metastases compared with primary high and low grade prostate cancers. Validation experiments using TaqMan® Low Density arrays confirmed this downregulation. Furthermore, SFRP2 was found to be downregulated in all four groups of prostate cancer compared with normal prostate. Therefore, SFRP2 has diagnostic potential.

Using clinical follow-up data, differences were observed in SFRP2 expression between the patients with curative treatment, biochemical recurrence after initial therapy and progressive disease. More than 50% of metastases showed a large downregulation of SFRP2. Moreover, also a few CRPC patients showed a very low SFRP2 expression. Therefore, SFRP2 can be used for the detection of patients with progression under endocrine therapy (CRPC) and patients with prostate cancer metastasis. It is therefore suggested, that in combination with a marker that is upregulated in metastases, a ratio of that marker and SFRP2 could be used for the detection of circulating tumor cells.

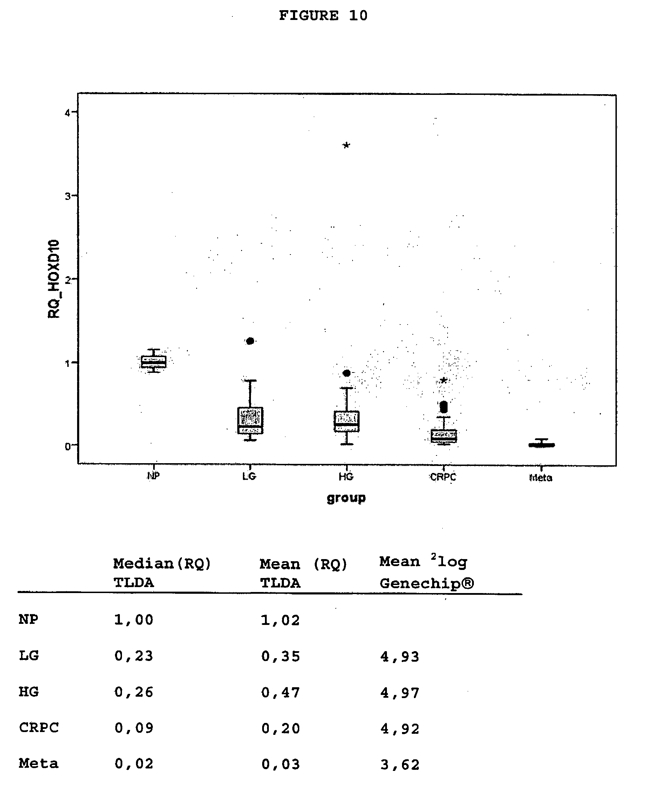

HOXD10 (**Figure 10**): The present GeneChip® Human Exon 1.0 ST Array data showed that HOXD10 was downregulated in prostate cancer metastases compared with primary high and low grade prostate cancers. Validation experiments using TaqMan® Low Density arrays confirmed this downregulation. Furthermore, HOXD10 was found to be downregulated in all four groups of prostate cancer compared with normal prostate. Therefore, HOXD10 has diagnostic potential.

Using clinical follow-up data, differences were observed in HOXD10 expression between the patients with curative treatment, biochemical recurrence after initial therapy and progressive disease. All metastases showed a large downregulation of HOXD10. Moreover, also a few CRPC patients showed a low HOXD10 expression. Therefore, HOXD10 can be used for the detection of patients with progression under endocrine therapy (CRPC) and patients with prostate cancer metastases.

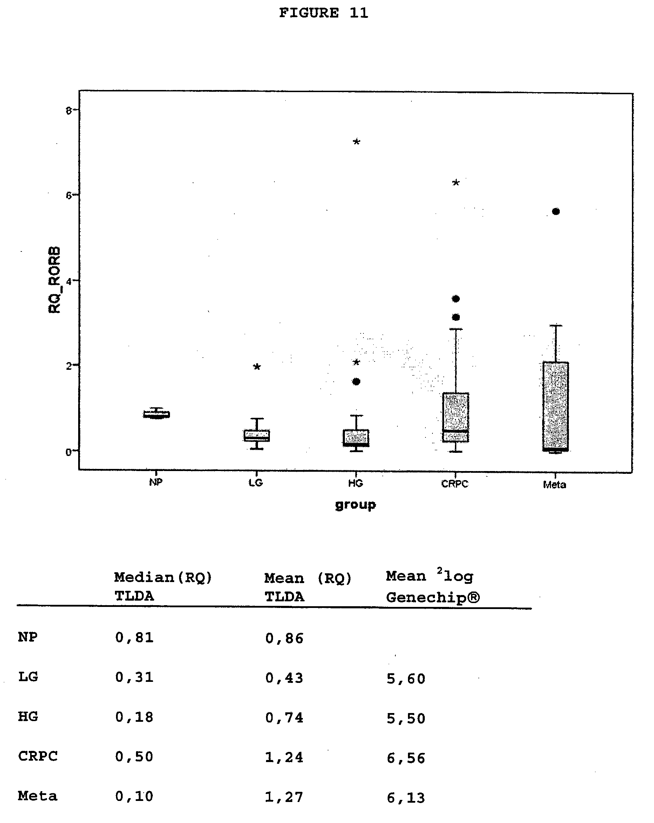

RORB (**Figure 11**): The present GeneChip® Human Exon 1.0 ST Array data showed that RORB was upregulated in prostate cancer metastases and CRPC compared with primary high and low grade prostate cancers. Validation experiments using TaqMan® Low Density arrays confirmed this upregulation. Furthermore, RORB was found to be downregulated in all low and high grade prostate cancers compared with normal prostate. In CRPC and metastases RORB is re-expressed at the level of normal prostate. Therefore, RORB has diagnostic potential.

Using clinical follow-up data, differences were observed in RORB expression between the patients with curative treatment, biochemical recurrence after initial therapy and progressive disease. However, in a number of cases in the CRPC and metastases the upregulation of RORB coincides with a downregulation of SFRP2. Using a ratio of RORB over SFRP2 could detect 75% of prostate cancer metastases. Furthermore, a number of CRPC patients had a high RORB/SFRP2 ratio. Therefore, this ratio can be used in the detection of patients with circulating tumor cells and progressive patients under CRPC.

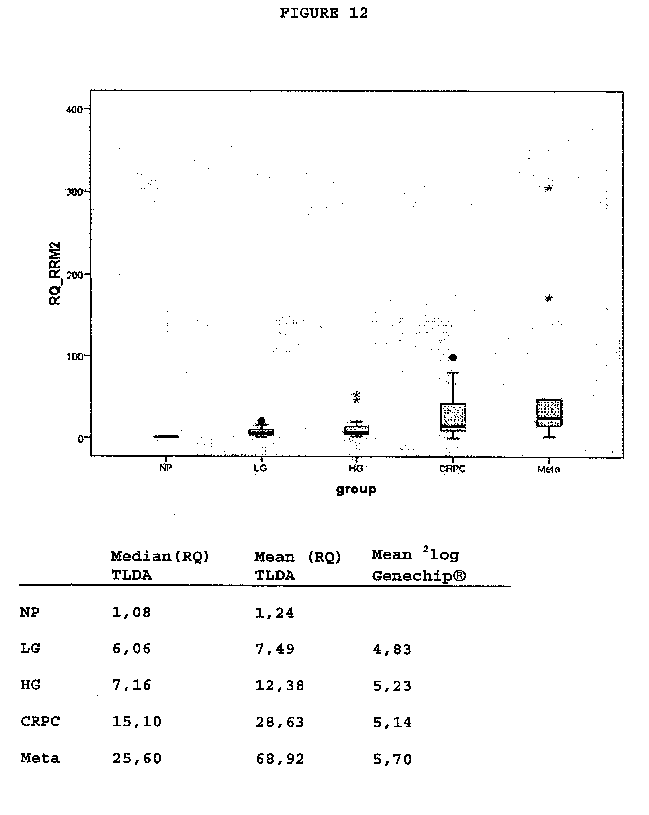

RRM2 (**Figure 12**): Experiments using TaqMan® Low Density arrays showed upregulation of RRM2 in all four groups of prostate cancer compared with normal prostate. Therefore, RRM2 has diagnostic potential. Moreover, the expression of RRM2 is higher in CRPC and metastasis showing that it may be involved in the invasive and metastatic potential of prostate cancer cells. Therefore, RRM2 can be used for the detection of circulating prostate tumor cells.

Using clinical follow-up data, differences were observed in RRM2 expression between the patients with curative treatment, biochemical recurrence after initial therapy and progressive disease.

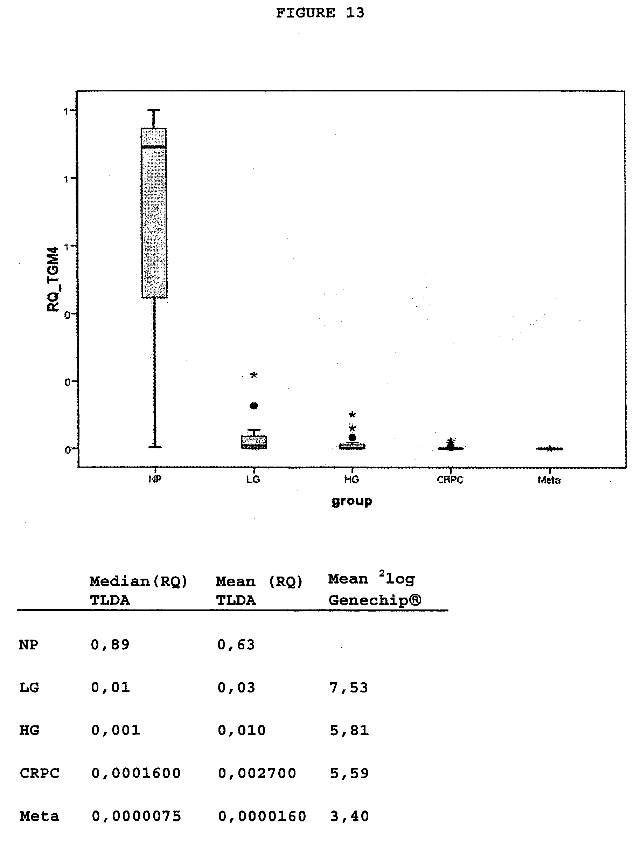

TGM4 (**Figure 13**): The present GeneChip® Human Exon 1.0 ST Array data showed that TGM4 was downregulated in prostate cancer metastases compared with primary high and low grade prostate cancers. Validation experiments using TaqMan® Low Density arrays confirmed this downregulation. Furthermore, TGM4 was found to be extremely downregulated in all four groups of prostate cancer compared with normal prostate. Therefore, TGM4 has diagnostic potential.

Using clinical follow-up data, it was observed that patients with progressive disease showed a stronger downregulation of TGM4 (subgroup of patients) compared with patients with curative treatment and biochemical recurrence after initial therapy. In metastases the TGM4 expression is completely downregulated. Therefore, TGM4 has prognostic potential.

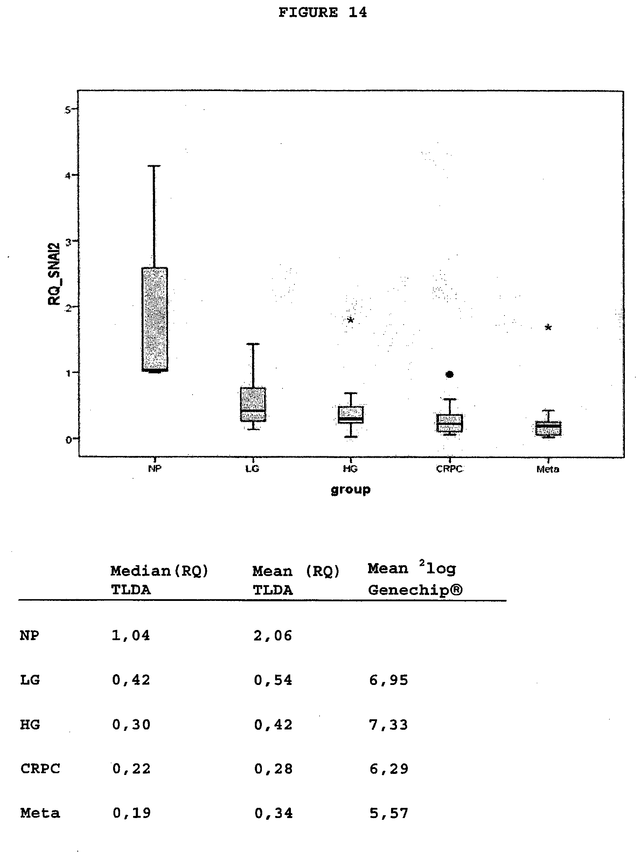

SNAI2 (**Figure 14**): The present GeneChip® Human Exon 1.0 ST Array data showed that SNAI2 was downregulated in prostate cancer metastases compared with primary high and low grade prostate cancers. Validation experiments using TaqMan® Low Density arrays confirmed this downregulation. Furthermore, SNAI2 was found to be downregulated in all four groups of prostate cancer compared with normal prostate. Therefore, SNAI2 has diagnostic potential.

Using clinical follow-up data, differences were observed in SNAI2 expression between the patients with curative treatment, biochemical recurrence after initial therapy and progressive disease.

## Claims

1. Method for establishing the presence, or absence, of prostate cancer in a human individual comprising:
a) determining the expression of the RRM2 gene in a sample originating from said human individual wherein determining the expression comprises determining mRNA expression;
b) establishing up-regulation of expression of said RRM2 gene as compared to expression of said RRM2 gene in a sample originating from said human individual not comprising prostate tumour cells or prostate tumour tissue, or from an individual not suffering from prostate cancer; and
c) establishing the presence, or absence, of prostate cancer based on the established up-regulation of said RRM2 gene.

2. Method according to claim 1, wherein said method further comprises:
d) determining the expression of one or more genes chosen from the group consisting of HOXC6, TGM4, RORB, HOXD10, SFRP2, and SNAI2 in said sample originating from said human individual;
e) establishing up, or down, regulation of expression of said one or more genes as compared to expression of said respective one or more genes in a sample originating from said human individual not comprising prostate tumour cells or prostate tumour tissue, or from an individual not suffering from prostate cancer; and
f) establishing the presence, or absence, of prostate cancer based on the established up-or down regulation of said one or more genes.

3. Method according to claim 1 or claim 2, wherein said one or more is selected from the group consisting of two or more; three or more; four or more; five or more and six.

4. Method according to any of the claims 1 to 3, wherein establishing the presence, or absence, of prostate cancer in a human individual further comprises identification, or diagnosing, low grade prostate cancer (LG), high grade prostate cancer (HG), prostate cancer with metastasis (PrCa Met) and/or castration resistant prostate cancer (CRPC), preferably castration resistant prostate cancer (CRPC).

## Patentansprüche

1. Verfahren zum Ermitteln des Vorhandenseins oder der Abwesenheit von Prostatakrebs in einem menschlichen Individuum, umfassend:
a) Bestimmen der Expression des RRM2-Gens in einer Probe, die von dem menschlichen Individuum stammt, wobei das Bestimmen der Expression umfasst, mRNA-Expression zu bestimmen;
b) Ermitteln einer Hinaufregulierung der Expression des RRM2-Gens verglichen mit der Expression des RRM2-Gens in einer Probe, die von dem menschlichen Individuum, umfassend keine Prostatatumorzellen oder kein Prostatatumorwebe, oder von einem Individuum, das nicht an Prostatakrebs leidet, stammt und
c) Ermitteln des Vorhandenseins oder der Abwesenheit von Prostatakrebs basierend auf der ermittelten Hinaufregulierung des RRM2-Gens.

2. Verfahren nach Anspruch 1, wobei das Verfahren des Weiteren umfasst:
d) Bestimmen der Expression von einem oder mehr Genen, ausgewählt aus der Gruppe bestehend aus HOXC6, TGM4, RORB, HOXD10, SFRP2 und SNAI2, in der Probe, die von dem menschlichen Individuum stammt;
e) Ermitteln einer Hinauf- oder Hinunterregulierung der Expression von dem bzw. den einen oder mehr Genen verglichen mit der Expression des bzw. der entsprechenden einen oder mehr Gene in einer Probe, die von dem menschlichen Individuum, umfassend keine Prostatatumorzellen oder kein Prostatatumorwebe, oder von einem Individuum, das nicht an Prostatakrebs leidet, stammt; und
f) Ermitteln des Vorhandenseins oder der Abwesenheit von Prostatakrebs basierend auf der ermittelten Hinauf- oder Hinunterregulierung des bzw. der einen oder mehr Gene.

3. Verfahren nach Anspruch 1 oder Anspruch 2, wobei das eine oder mehr ausgewählt ist aus der Gruppe bestehend aus zwei oder mehr, drei oder mehr, vier oder mehr, fünf oder mehr und sechs.

4. Verfahren nach einem der Ansprüche 1 bis 3, wobei das Ermitteln des Vorhandenseins oder der Abwesenheit von Prostatakrebs in einem menschlichen Individuum des Weiteren ein Identifizieren oder Diagnostizieren von geringgradigem Prostatakrebs ('low grade'; LG), hochgradigem Prostatakrebs ('high grade'; HG), Prostatakrebs mit Metastasierung (PrCa Met) und/oder kastrationsresistentem Prostatakrebs (CRPC), vorzugsweise kastrationsresistentem Prostatakrebs (CRPC) umfasst.

## Revendications

1. Procédé d'établissement de la présence, ou de l'absence, du cancer de la prostate sur un individu humain comprenant :
a) la détermination de l'expression du gène RRM2 dans un échantillon provenant dudit individu humain dans lequel la détermination de l'expression comprend la détermination de l'expression de l'ARN m ;
b) l'établissement d'une régulation positive de l'expression dudit gène RRM2 par comparaison avec l'expression dudit gène RRM2 dans un échantillon provenant dudit individu humain ne comportant pas de cellules de tumeur de la prostate ou de tissu de tumeur de la prostate, ou d'un individu ne souffrant pas du cancer de la prostate ; et
c) l'établissement de la présence, ou de l'absence, du cancer de la prostate sur la base de la régulation positive établie dudit gène RRM2.

2. Procédé selon la revendication 1, dans lequel ledit procédé comprend en outre :
d) la détermination de l'expression d'un ou plusieurs gènes sélectionnés à partir du groupe constitué par HOXC6, TGM4, RORB, HOXD10, SFRP2, et SNAI2 dans ledit échantillon provenant dudit individu humain ;
e) l'établissement d'une régulation positive ou négative de l'expression desdits un ou plusieurs gènes par comparaison à l'expression dudit un ou plusieurs gènes respectifs dans un échantillon provenant dudit individu humain ne comportant pas de cellules de tumeur de la prostate ou de tissu de tumeur de prostate, ou d'un individu ne souffrant pas du cancer de la prostate ; et
f) l'établissement de la présence, ou de l'absence, du cancer de la prostate sur la base de la régulation positive ou négative établie desdits un ou plusieurs gènes.

3. Procédé selon la revendication 1 ou 2, dans lequel lesdits un ou plusieurs sont sélectionnés parmi le groupe constitué par deux ou plus ; trois ou plus ; quatre ou plus ; cinq ou plus et six.

4. Procédé selon l'une quelconque des revendications 1 à 3, dans lequel l'établissement de la présence, ou de l'absence, du cancer de la prostate sur un individu humain comprend en outre l'identification, ou le diagnostic, d'un cancer de la prostate de faible grade (LG), d'un cancer de la prostate de grade élevé (HG), d'un cancer de la prostate avec métastases (PrCa Met) et/ou d'un cancer de la prostate résistant à la castration (CRPC), de préférence, d'un cancer de la prostate résistant à la castration (CRPC).
